# EUROPEAN PATENT APPLICATION

(11) **EP 1 709 922 A1**
(43) Date of publication of application: **11.10.2006**
(21) Application number: 04807410.8
(22) Date of filing: 21.12.2004
(51) Int. Cl.: A61B 18/12

(54) **BALLOON CATHETER**

(30) Priority: 06.01.2004 JP 2004000874; 24.02.2004 JP 2004047730
(71) Applicant: TORAY INDUSTRIES, INC., Tokyo 103-8666 (JP)
(72) Inventor: MATSUKUMA, Akinori, .. (JP); YAMAZAKI, Yoshiharu, .. (JP)
(74) Representative: Schaeberle, Steffen
(86) International application number: PCT/JP2004/019053
(87) International publication number: WO 2005/065559

(57) **Abstract**

A balloon catheter in which a high-frequency current is supplied between electrodes spaced in a balloon, the liquid in the balloon is heated, and the heat conducted through the balloon ablates the organism tissue in contact with the balloon, **characterized in that** the area of the surface of each electrode is 20 mm² or more or a potential detecting electrode for detecting the potential at an abrasion portion is disposed outside the balloon and in a position at least before or behind the balloon.

## Description

### Technical field

The present invention relates to a balloon catheter. In more detail, the invention relates to a balloon catheter to be inserted into a patient's body, for keeping the balloon of the catheter in contact with a target lesion site with an intention to heat the target lesion site, through the balloon, by the heat of the liquid internally filling the balloon heated by the high-frequency dielectric heating and the Joule heating respectively caused by high-frequency current, in order to perform the ablation of the target lesion site by means of the heat. This catheter is called a balloon ablation catheter.

### Background art

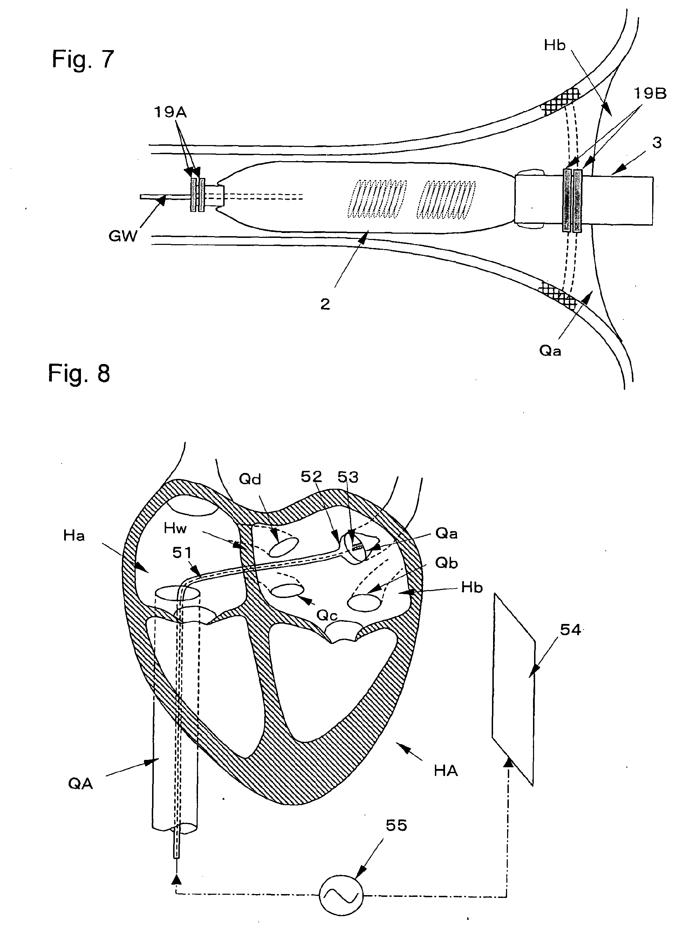

Ablation catheters for use in treating of cardiac arrhythmia are developed. Patent document 1 describes a balloon ablation catheter to electrically isolate pulmonary veins for treating of arrhythmia. In the case where such a balloon ablation catheter is used for electrically isolating pulmonary veins, as shown in Fig. 8, an inflatable/deflatable balloon 52 disposed at the distal end of a catheter 51 is percutaneously introduced into the inferior vena cava QA, and the catheter 51 is used to press the balloon 52 for letting it from the right atrium Ha of the heart HA into the left atrium Hb through the interatrial septum Hw. Then, a liquid containing a contrast medium is supplied into the balloon 52, to inflate it, for applying and keeping the balloon 52 to and wedging into the pulmonary vein ostium Qa. A high-frequency coil electrode 53 formed as a coil by spirally winding a cross-sectionally completely round electric wire having a diameter of about 0.5 mm is disposed in the balloon 52. High-frequency power is supplied from a high-frequency current source 55 to the high-frequency coil electrode 53, and high-frequency energization is performed between the high-frequency coil electrode 53 and a high-frequency external electrode (hereinafter called the counter electrode plate) 54 disposed the patient's body surface.

The heat generated as the high-frequency dielectric heating and the Joule heating respectively caused by the high-frequency energization between the high-frequency coil electrode 53 and the counter electrode plate 54 allows the annularly circumferential general ablation of the pulmonary vein ostium Qa. In succession to the ablation of the pulmonary vein ostium Qa, the ablation of the remaining three pulmonary vein ostia Qb, Qc and Qd respectively open within the inner wall of the left atrium Hb is similarly performed one after another.

Since the annularly circumferential ablation of the respective pulmonary vein ostia Qa to Qd is performed, all the four pulmonary veins are electrically isolated. If all the four pulmonary veins are electrically isolated by the annularly circumferential ablation of the respective pulmonary vein ostia Qa to Qd, the electric signals causing the arrhythmia are intercepted and the arrhythmia is virtually cured.

If the balloon ablation catheter described in patent document 1 as described above is used, the annularly circumferential general ablation of the respective pulmonary vein ostia Qa to Qd can be performed. So, it is not necessary to repeat ablation. Furthermore, since the ablation is performed only at the annular circumferences of the pulmonary vein ostia Qa to Qd, the ablation at any unnecessary portion (for example, healthy portion) can be avoided.

However, in the case of the balloon ablation catheter using the counter electrode plate, the high-frequency electrical current during ablation may cause the counter electrode plate 54 attached to the patient's body surface to generate heat.

Furthermore, a guide wire is necessary to introduce the balloon ablation catheter into the target lesion site in a patient's body. So, if a metal coil type guide wire or a guide wire having a thin plastic covering is used in the balloon ablation catheter with the counter electrode plate, the high-frequency power supply during ablation causes the high-frequency electrical current to flow also to the tip of the guide wire. As a result, the tip of the guide wire is also heated, and the ablation of a blood vessel or tissue other than the target lesion site may also be performed.
Furthermore, after the aforesaid balloon ablation catheter has finished the intended ablation, it is pull out, and another catheter for potential detection (not shown in the drawing) is inserted to the ablation site for detecting the potentials at and around the ablation site. This is necessary to check whether or not the ablation has been performed adequately and whether or not the electric isolation has been achieved. In the case where the ablation has not been adequately performed, the insertion and removal of the balloon ablation catheter and the potential detecting catheter must be repeated.

To avoid this complicated work, it can be considered to let the balloon ablation catheter have a potential detecting means. However, in the case of a balloon ablation catheter using a counter electrode plate as described in patent document 1, the high-frequency power supply during ablation causes the high-frequency electrical current to flow also in the potential detecting electrodes, to heat the potential detecting electrodes, and thereby ablation may be caused also at a blood vessel or tissue other than the target lesion site.

Another means for heating the inside of the balloon is the method described in patent document 2. Patent document 2 discloses a medical system (200) (this number is stated in patent document 2; hereinafter this applies in this paragraph) comprising a balloon catheter with a sharp distal end. This system has two high-frequency electrodes (22 and 24) disposed in a balloon (8) as a means for heating the liquid (36) supplied into the balloon (8) . During a medical procedure, the balloon is kept in a deflated state, and the sharp distal end is used to puncture the organ to be cured, for letting the balloon reach the treating site. Then, the liquid (36) is supplied into the balloon (8), to inflate the balloon (8). In this state, high-frequency power is supplied across the high-frequency electrodes (22 and 24) . The high frequency dielectric heating and the Joule heating respectively caused by the high-frequency current between the high-frequency electrodes (22 and 24) heat the fluid (36). As a result, the undesired cells in the organism are heated through the balloon (8), and destroyed. The target tissue can be a malignant or benign tumor, cyst, or exogenously formed tissue narrowing a nearby body cavity.

However, the medical system described in patent document 2 merely causes general necrosis of the cells at and near the punctured portion by heating, and cannot be used for delicate and fine operation like the electrical isolation of pulmonary veins. Furthermore, the medical system has a problem that the liquid in the balloon may boil depending on the forms of the two high-frequency electrodes disposed in the balloon and the distance between the electrodes.
Patent document 1: JP 2002-78809 A
Patent document 2: JP 10-503407 A

### Disclosure of the invention

### Problem to be solved by the invention

The problem to be solved by the invention is to provide a balloon catheter (balloon ablation catheter) that can avoid the injury of the body surface by use of the counter electrode plate and the ablation at an area other than the target lesion site, can prevent the boiling of the liquid in the balloon, and can also avoid the repeated insertion and removal of the balloon ablation catheter and the potential detecting catheter.

### Means for solving the problem

A balloon catheter of the invention comprises a catheter shaft, a balloon attached to the catheter shaft, a first electrode and a second electrode positioned in the balloon with a clearance kept between them along the catheter shaft, high-frequency power supply leads for supplying high-frequency power between the first and second electrodes, and a liquid supply passage for supplying a liquid into the balloon, wherein the surface area SA of the first electrode and the surface area SB of the second electrode are 20 mm² or more respectively.

A balloon catheter of the invention comprises a catheter shaft, a balloon attached to.the catheter shaft, a first electrode and a second electrode positioned in the balloon with a clearance kept between them along the catheter shaft, high-frequency power supply leads for supplying high-frequency power between the first and second electrodes, and a liquid supply passage for supplying a liquid into the balloon, wherein potential detecting electrodes for detecting the potentials of the therapeutic site are disposed on the catheter shaft outside the balloon on the front end side or rear end side of the catheter shaft, and potential information deriving leads for deriving the potential information detected by the potential detecting electrodes are provided. In this balloon catheter of the invention, it is preferred that the surface area SA of the first electrode and the surface area SB of the second electrode are 20 mm² or more respectively.

In the balloon catheter of the invention, it is preferred that the shortest distance Esd between the first electrode and the second electrode is 1 mm or more.

In the balloon catheter of the invention, it is preferred that a spacer for keeping the clearance between the first electrode and the second electrode is disposed between these electrodes.

It is preferred that the balloon catheter of the invention further comprises a temperature sensor disposed inside or on the outer surface of the balloon, and temperature information deriving leads for deriving the temperature information detected by the temperature sensor.

In the balloon catheter of the invention, it is preferred that the catheter shaft comprises an outer cylindrical shaft and an inner cylindrical shaft provided in the outer cylindrical shaft movably along the outer cylindrical shaft; that the front end of the balloon is fixed to the front end of the inner cylindrical shaft while the rear end of the balloon is fixed to the front end of the outer cylindrical shaft, so that when the inner cylindrical shaft is moved relatively to the outer cylindrical shaft, the balloon can be deformed; and that the first and second electrodes are positioned with a clearance kept between them along the inner cylindrical shaft.

In the balloon catheter of the invention, it is preferred that the catheter shaft comprises an outer cylindrical shaft and an inner cylindrical shaft provided in the outer cylindrical shaft movably along the outer cylindrical shaft; that the front end of the balloon is fixed to the front end of the inner cylindrical shaft while the rear end of the balloon is fixed to the front end of the outer cylindrical shaft, so that when the inner cylindrical shaft is moved relatively to the outer cylindrical shaft, the balloon can be deformed; that the first and second electrodes are positioned with a clearance kept between them along the inner cylindrical shaft; that in the case where the potential detecting electrodes are positioned outside the balloon on the front end side of the catheter shaft, the potential detecting electrodes are installed on the inner cylindrical shaft; and that in the case where the potential detecting electrodes are positioned outside the balloon on the rear end side of the catheter shaft, the potential detecting electrodes are disposed on the outer cylindrical shaft.

In the balloon catheter of the invention, it is preferred that the liquid supply passage is formed as the clearance between the outer cylindrical shaft and the inner cylindrical shaft.

In the balloon catheter of the invention, it is preferred that a temperature information processor connected with the temperature information deriving leads and a high-frequency power adjusting device connected with the high-frequency power supply leads are provided to ensure that the high-frequency power supplied to the first and second electrodes can be adjusted by the high-frequency power adjusting device in response to the temperature judged by the temperature information processor.

In the balloon catheter of the invention, it is preferred that the frequency of the high-frequency power supplied to the first and second electrodes is 100 KHz to 2.45 GHz, and that the high-frequency power heats the liquid supplied from the liquid supply passage into the balloon for filling the balloon, to a temperature of 50°C to 80°C.

In the balloon catheter of the invention, it is preferred that a liquid agitator connected with the liquid supply passage is provided to ensure that the liquid supplied from the liquid supply passage into the balloon for filling the balloon can be reciprocated between the liquid supply passage and the inside of the balloon so that the liquid can be agitated in the balloon.

### Effects of the invention

This invention provides a balloon ablation catheter free from the possibility that the counter electrode generates heat, since both the high-frequency electrodes are disposed in the balloon, to get rid of the conventional counter electrode disposed outside a patient's body.

Since both the high-frequency electrodes are placed in the balloon made of an electrically highly resistant material, it does not happen that high-frequency electrical current flows to the tip of the guide wire during ablation. Therefore, the invention provides a balloon ablation catheter, in which the ablation of a blood vessel or tissue other than the target lesion site by the heating at the tip of the guide wire does not occur.

If the surface areas of both the high-frequency electrodes are 20 mm² or more respectively, and in addition, preferably, if the shortest distance between the electrodes is 1 mm or more, then the invention provides a balloon ablation catheter that allows the temperature in the balloon to be raised without causing the liquid in the balloon to boil.

If a temperature sensor is disposed inside or on the outer surface of the balloon, the invention provides a balloon ablation catheter that allows the temperature of the inside or surface of the balloon to be accurately detected.

If a spacer is installed between both the high-frequency electrodes, it does not happen that the high-frequency electrodes approach each other during the insertion of the balloon catheter into a patient's body or during a medical procedure, and such problems that the liquid near the high-frequency electrodes boils and that the high-frequency electrodes are short-circuited not to allow heating can be avoided. Thus, the invention provides a balloon ablation catheter in which the temperature in the balloon can be stably controlled.

If potential detecting electrodes for detecting the potentials near the ablation site are disposed on the catheter shaft outside the balloon on the front end side or the rear end side of the catheter shaft, the potential detecting electrodes can be used to detect the potentials near the therapeutic ablation site after completion of an ablation process at the target lesion site, for judging whether or the ablation has been adequate, without taking out the balloon catheter. Furthermore, if the ablation has been found to be inadequate as a result of judgment, the balloon can be immediately inflated again to repeat the ablation process . As a result, it is not necessary to insert the potential detecting catheter or to insert the balloon ablation catheter again. The patient can be liberated from the burden of invasion arising from the insertion of the potential detecting catheter and the re-insertion of the balloon ablation catheter. Therefore, the invention provides a balloon ablation catheter that allows the burden caused by the invasion of catheters on the patient to be reduced.

Since both the high-frequency electrodes are disposed in the balloon made of an electrically highly resistant material, it does not happen that high-frequency electrical current flows to the potential detecting electrodes during ablation. Therefore, the invention provides a balloon ablation catheter, in which the ablation of a blood vessel or tissue other than the target lesion site by the heating of the potential detecting electrodes does not occur.

The balloon catheter (balloon ablation catheter) of the invention allows an annularly wide range of ablation to be performed along the full circumference of the balloon in one ablation process. Therefore, it is not necessary to specify individual abnormal portions of ablation as done so far. It is only required to judge whether or not there is any abnormality at the ablation site, i.e., whether or not a predetermined potential has been detected. If there is any abnormality, it is only required to perform another ablation process at the site. It is not necessary to dispose many potential detecting electrodes in the catheter as done so far. Furthermore, since it is not necessary to specify abnormal portions, it is not necessary to keep potential detecting electrodes in contact with specific portions as done so far. It is only required to position potential detecting electrodes near the site of annular ablation. As a result, the number of expensive potential detecting electrodes to be disposed can be decreased, and the invention provides a low-cost and small-sized balloon ablation catheter.

The catheter shaft can comprise an outer cylindrical shaft and an inner cylindrical shaft, and the inner cylindrical shaft can be moved in the axial direction of the outer cylindrical shaft, to variously change the form of the balloon. Furthermore, since both the high-frequency electrodes are fitted around the inner cylindrical shaft concentrically, both the high-frequency electrodes can be substantially integrated with the inner cylindrical shaft. As a result, the invention provides a balloon ablation catheter that can be smoothly inserted into a patient's body.

If a temperature information processor connected with the temperature information deriving leads and a high-frequency power adjusting device connected with the high-frequency power supply leads are provided, the high-frequency power can be supplied quantitatively in response to the temperature found by the temperature sensor. As a result, the invention provides a balloon ablation catheter in which the heating temperature by high-frequency dielectric heating and Joule heating can be accurately controlled.

If a liquid agitator connected with the liquid supply passage is provided, the liquid in the balloon inflated by the liquid introduced in it can be reciprocated between the liquid supply passage and the inside of the balloon during the heating by high frequency dielectric heating and Joule heating. Thus, the invention can provide a balloon ablation catheter in which the liquid in the balloon is agitated to mix liquid portions different in temperature for uniforming the liquid temperature in the balloon, thereby lessening the heating irregularity caused by high-frequency dielectric heating and Joule heating.

### Brief description of the drawings

Fig. 1 is a schematic side view showing an embodiment of the balloon catheter of the invention.
Fig. 2 is a longitudinal sectional view showing the balloon and its vicinity of the balloon catheter shown in Fig. 1.
Fig. 3 is a longitudinal sectional view showing an external form of an inflated balloon of the balloon catheter shown in Fig. 1.
Fig. 4 is a sectional view of the balloon catheter shown in Fig. 2 along the X-X arrow.
Fig. 5 is a typical side view showing a state where the ablation of a pulmonary vein opening is performed by the balloon catheter shown in Fig. 1.
Fig. 6 is a typical side view showing a state where the potentials of the therapeutic site are detected by the potential detecting electrodes disposed on the front end side of the balloon catheter shown in Fig. 1.
Fig. 7 is a typical side view showing a state where the potentials of the therapeutic site are detected by the potential detecting electrodes installed on the rear end side of the balloon catheter shown in Fig. 1.
Fig. 8 is a typical vertical sectional view for illustrating a state where the ablation of pulmonary vein openings is performed by the conventional balloon ablation catheter with a counter electrode plate placed outside a patient's body.

### Meanings of symbols

- 1:: balloon catheter (balloon ablation catheter)
- 2:: balloon
- 2A:: liquid introducing port
- 2R:: rear end of balloon
- 2F:: front end of balloon
- 3:: outer cylindrical shaft
- 3A:: metallic pipe
- 3B:: support
- 3F:: front end of outer cylindrical shaft
- 4:: inner cylindrical shaft
- 4A:: metallic pipe
- 4F:: front end of inner cylindrical shaft
- 5A:: first electrode (high-frequency electrode)
- 5B:: second electrode (high-frequency electrode)
- 6:: liquid supply device
- 6A:: liquid supply passage
- 7:: four-way connector
- 8:: liquid agitator
- 9:: temperature sensor
- 10:: high-frequency power supply apparatus
- 11:: temperature information deriving lead
- 12A,: 12B: high-frequency power supply lead
- 13:: electrically insulating protective film
- 14:: electrically insulating protective film
- 15:: synthetic resin pipe
- 17:: spacer
- 18:: electrically insulating protective covering
- 19A, 19B:: potential detecting electrode
- 20A, 20B:: potential information deriving lead
- 21:: electrocardiograph
- 51:: catheter
- 52:: balloon
- 53:: high-frequency coil electrode
- 54:: high-frequency external electrode (counter electrode)
- 55:: high-frequency current source
- CS:: catheter shaft
- Esd:: shortest distance between high-frequency electrodes

- GW:: guide wire
- HA:: heart
- Ha:: right atrium
- Hb:: left atrium
- Hw:: interatrial septum
- QA:: inferior vena cava
- Qa, Qb, Qc, Qd:: pulmonary vein ostium
- SA:: surface area of first electrode
- SB:: surface area of second electrode
The best modes for carrying out the invention

This invention is explained below in more detail based on an embodiment.

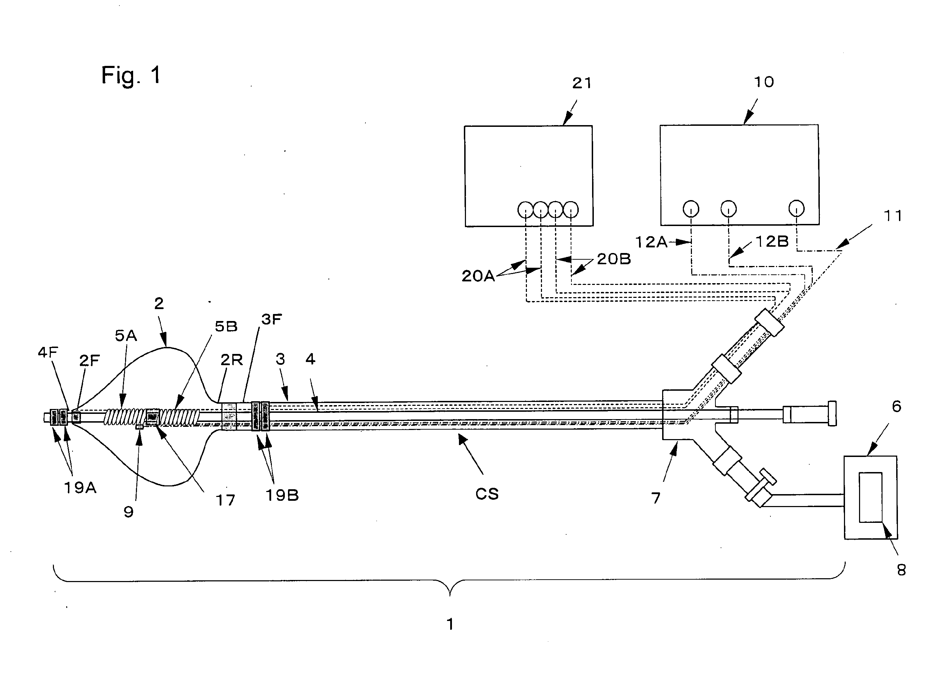

In Fig. 1, the balloon catheter (balloon ablation catheter) 1 of the invention has a catheter shaft CS. The catheter shaft CS comprises an outer cylindrical shaft 3 and an inner cylindrical shaft 4 provided inside the outer cylindrical shaft 3 movably along the outer cylindrical shaft 3.

The balloon catheter 1 has a balloon 2 attached to it. The balloon 2 can be deformed and is made of an electrically highly resistant material capable of being inflated and deflated. The front end 2F of the balloon 2 is fixed to the front end 4F of the inner cylindrical shaft 4, and the rear end 2R of the balloon 2 is fixed to the front end 3F of the outer cylindrical shaft 3.

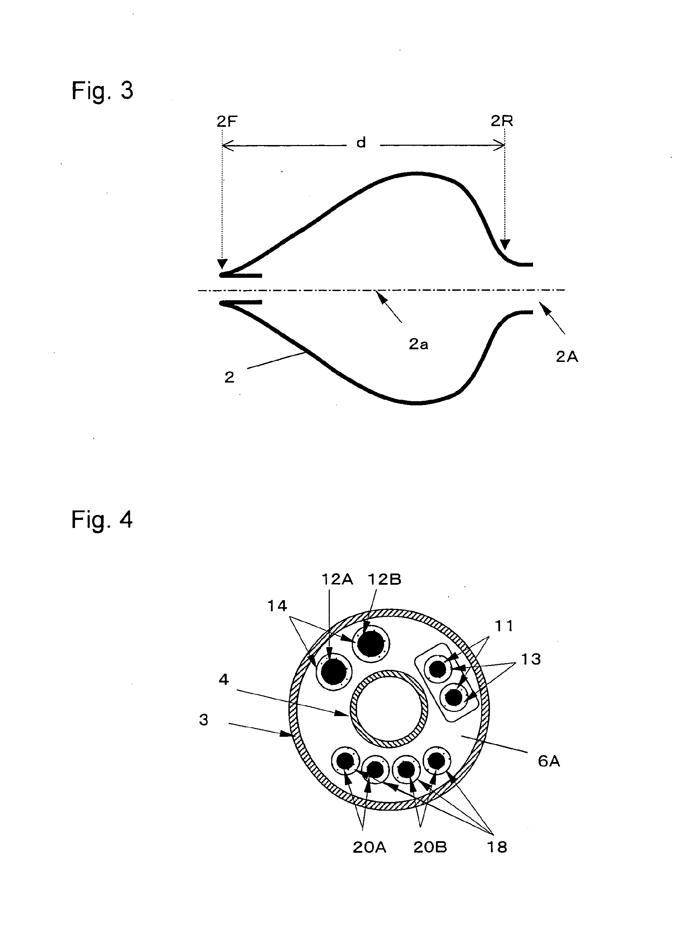

The balloon catheter 1 has a first electrode 5A and a second electrode 5B positioned in the balloon 2 with a clearance kept between them along the inner cylindrical shaft 4. The first electrode 5A and the second electrode 5B may also be respectively called a high-frequency electrode 5A and a high-frequency electrode 5B hereinafter. High-frequency power supply lead 12A (Fig. 4) for supplying high-frequency power is connected with the first electrode 5A, and high-frequency power supply lead 12B (Fig. 4) for supplying high-frequency power is connected with the second electrode 5B.

The balloon catheter 1 has a liquid supply passage 6A (Fig. 4) for supplying a liquid into the balloon 2. The liquid supply passage 6A is formed as the clearance between the outer cylindrical shaft 3 and the inner cylindrical shaft 4. The rear end 2R of the balloon 2 has a liquid introducing port 2A (Fig. 3) communicating with the liquid supply passage 6A.

In the balloon catheter 1, the surface area SA of the first electrode 5A is 20 mm² or more, and the surface area SB of the second electrode 5B is also 20 mm² or more.

In the balloon catheter 1, potential detecting electrodes 19A for detecting the potentials of the therapeutic site are installed on the inner cylindrical shaft 4 outside the balloon 2 on the front end side of the catheter shaft CS, and potential detecting electrodes 19B for detecting the potentials of the therapeutic site are installed on the outer cylindrical shaft 3 outside the balloon 2 on the rear end side of the catheter shaft CS. Potential information deriving leads 20A (Fig. 4) for deriving the potential information detected by the potential detecting electrodes 19A are connected with the potential detecting electrodes 19A, and potential information deriving leads 20B (Fig. 4) for deriving the potential information detected by the potential detecting electrodes 19B are connected with the potential detecting electrodes 19B.

At the proximal end of the balloon catheter 1, a four-way connector 7 for supporting the outer cylindrical shaft 3 and the inner cylindrical shaft 4 is attached. The liquid supply passage 6A is connected with a liquid supply device 6 through the four-way connector 7. The high-frequency power supply leads 12A and 12B are connected with high-frequency power supply apparatus 10 through the four-way connector 7. The potential information deriving leads 20A and 20B are connected with an electrocardiograph 21 through the four-way connector 7.

The catheter shaft CS of the balloon catheter 1 of this embodiment is a double cylindrical catheter shaft comprising the outer cylindrical shaft 3 and the inner cylindrical shaft 4, and the outer cylindrical shaft 3 or the inner cylindrical shaft 4 can be moved in the axial direction to variously change the form of the balloon 2. Therefore, this is a preferred mode as a catheter shaft used for carrying out the invention. However, the catheter shaft used for carrying out the invention is not necessarily limited to a double cylindrical catheter shaft, and depending on the type of therapy, a single cylindrical catheter shaft can also be used.

The lengths of the outer cylindrical shaft 3 and the inner cylindrical shaft 4 are usually about 1 m to about 1.4 m. The outer diameter of the outer cylindrical shaft 3 is about 3 mm to about 5 mm, and the inner diameter of it is about 2 mm to about 4 mm. The outer diameter of the inner cylindrical shaft 4 is about 1 mm to about 3 mm, and the inner diameter of it is about 0.5 mm to about 2 mm.

The material of the outer cylindrical shaft 3 and the inner cylindrical shaft 4 is selected from highly anti-thrombogenic flexible materials. The materials include, for example, fluorine resins, polyamide resins and polyimide resins.

As shown in Fig. 3, the balloon 2 as inflated has a conical outer form smaller in diameter toward the front end 2F (like a tapered cone). The length d of the balloon 2 (the length along the central axis 2a virtually connecting the balloon front end 2F and the balloon rear end 2R) is about 20 mm to about 40 mm. The largest outer diameter on the rear end side 2R is about 10 mm to about 40 mm. The film thickness of the balloon 2 is 100 µm to 300 µm. In the case where the balloon 2 has an outer form like a tapered cone, it is prevented that the balloon 2 goes into a pulmonary vein. Furthermore, since the front end of the balloon 2 is slightly inserted into a pulmonary vein ostium, the balloon 2 tightly contacts the pulmonary vein ostium, to assure the annularly circumferential general ablation of the pulmonary vein ostium.

The material of the balloon 2 is selected from highly anti-thrombogenic elastic materials. Furthermore, it is desirable that the material of the balloon 2 is made of an electrically highly resistant material to prevent that the high-frequency electrical current leaks outside the balloon 2 in the case where high-frequency electrical current flows between the high-frequency electrodes 5A and 5B. As the material of the balloon 2, a polyurethane-based material is especially preferred. Particular examples of the material include thermoplastic polyether urethane, polyether polyurethane urea, fluorine polyether urethane urea, polyether polyurethane urea resin and polyether polyurethane urea amide.

As the high-frequency electrodes of the invention, it is important that both the high-frequency electrodes are positioned in the balloon 2 like the high-frequency electrodes 5A and 5B shown in Fig. 1.

Each of the high-frequency electrodes 5A and 5B shown in Fig. 1 is formed by winding an electric wire like a coil. However, the high-frequency electrodes are not limited to coils in form and can have any other form. However, cylindrical high-frequency electrodes formed like coils or cylinders are preferred.

In the invention, it is important that the surface areas SA and SB of the high-frequency electrodes are 20 mm² or more respectively. Preferred surface areas are 30 mm² or more, and more preferred surface areas are 40 mm² or more. It is preferred that the surface areas are 400 mm² or less.

When the electrode is formed like a cylindrical sheet, the surface area of the electrode refers to the total surface area including the area of the outer surface, the area of the inner surface and the area of both the end surfaces (area of the thickness portion). When the electrode is formed like a cylindrical coil, the surface area of the electrode can be approximated by the surface area of the electric wire forming the coil corresponding to the electrode portion.

It is preferred that the shortest distance Esd between the high-frequency electrodes is 1 mm or more. It is preferred that the shortest distance Esd between the high-frequency electrodes is 30 mm or less.

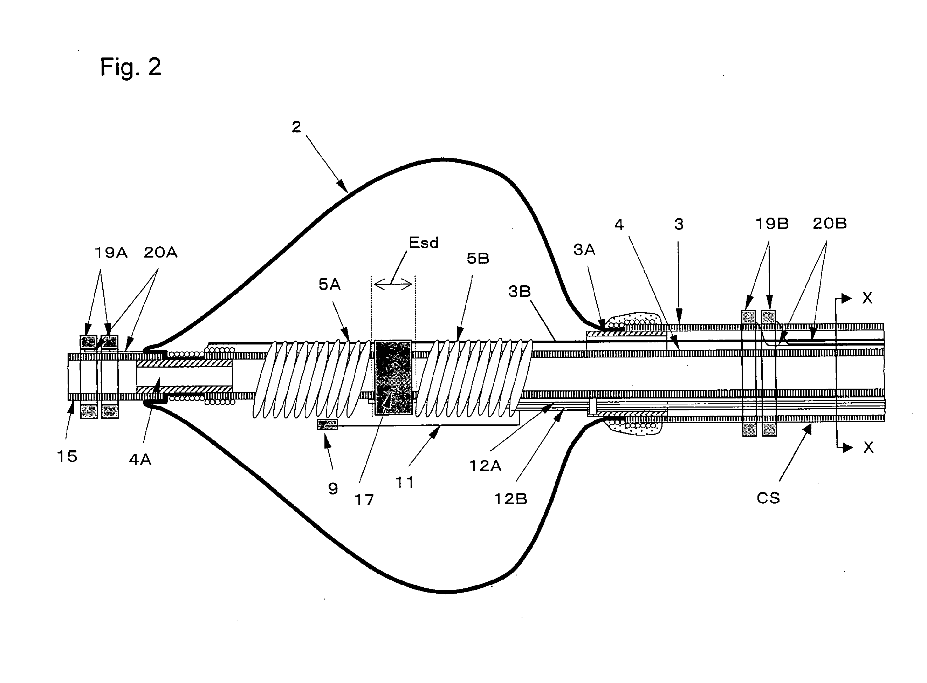

If the electrodes are formed like coils for example, the shortest distance Esd between the high-frequency electrodes refers to the straight distance connecting the mutually closest points of the high-frequency electrodes 5A and 5B as shown in Fig. 2.

If the surface areas SA and SB of the high-frequency electrodes and the shortest distance Esd between them are kept in the above-mentioned ranges, good heating efficiency can be obtained for the liquid in the balloon 2.

In the case where the high-frequency electrodes are formed like coils, the electric wires used are not especially limited in diameter. However, it is practically preferred that the diameter is about 0.1 mm to about 1 mm.

As the material of the high-frequency electrodes, a metal (wire) having a high electric conductivity such as silver (wire), gold (wire), platinum (wire) or copper (wire) can be used.

The high-frequency electrodes 5A and 5B are fitted concentrically around the inner cylindrical shaft 4 in such a manner that the electrodes do not curb the movement of the inner cylindrical shaft 4. The inner diameter of the high-frequency electrodes 5A and 5B is slightly larger than the outer diameter of the inner cylindrical shaft 4, and a slight clearance is formed between the inner surfaces of the high-frequency electrodes 5A and 5B and the outer surface of the inner cylindrical shaft 4.

If the high-frequency electrodes 5A and 5B are fitted concentrically around the inner cylindrical shaft as described above, the central axis of the high-frequency electrodes 5A and 5B automatically agrees with the central axis of the catheter 1, and in addition, the high-frequency electrodes 5A and 5B are substantially integrated with the inner cylindrical shaft 4. Furthermore, since the high-frequency electrodes 5A and 5B do not curb the movement of the inner cylindrical shaft 4, the inner cylindrical shaft 4 can move smoothly.

It is preferred that a spacer 17 is inserted between the high-frequency electrodes 5A and 5B to keep the shortest distance Esd between the high-frequency electrodes at 1 mm or more and to prevent that the shortest distance Esd becomes less than 1 mm during use. The form of the spacer 17 is not especially limited, but a cylindrical sheet with a diameter virtually equal to that of the high-frequency electrodes formed like coils is preferred. This spacer 17 is also fitted concentrically around the inner cylindrical shaft 4 in such a manner that it does not curb the movement of the inner cylindrical shaft 4 like the high-frequency electrodes 5A and 5B. In this constitution, the inner cylindrical shaft 4 can move smoothly.

In the balloon catheter 1, the spacer 17 and the high-frequency electrodes 5A and 5B are not connected with each other, but are positioned independently from each other. However, a mode in which the high-frequency electrodes 5A and 5B are bonded to both the ends of the spacer 17 by such a means as bonding or a mode in which either the high-frequency electrode 5A or 5B is bonded to one end of the spacer 17 can also be employed. Furthermore, in the case where the high-frequency electrodes 5A and 5B are formed like coils, a mode in which the high-frequency electrodes 5A and 5B are wound around the spacer 17 per se can also be employed. It is important that the distance between the high-frequency electrodes 5A and 5B is maintained by the spacer, for being prevented from becoming shorter than 1 mm.

The material of the spacer is a resin having low electric conductivity. Particular examples of the material include fluorine resins, polyamide resins and polyimide resins.

In the case where the balloon catheter 1 of the invention is used for treatment of a patient, the high-frequency electrical current needed for ablation flows between the high-frequency electrodes 5A and 5B in the balloon 2. As a result, the liquid in the balloon 2 is heated by high frequency dielectric heating and Joule heating. The adequate temperature for ablation of the tissue based on the heating by high frequency dielectric heating and Joule heating is usually in a range from 50°C to 70°C.

The liquid supply device 6 has a liquid feed roller pump (not shown in the drawings), and the liquid supplied by the liquid feed roller pump passes through the liquid supply passage 6A (Fig. 4) formed as a clearance between the outer cylindrical shaft 3 and the inner cylindrical shaft 4 and is supplied into the balloon 2 through the liquid introducing port 2A (Fig. 3) . As the liquid is supplied into the balloon 2, the balloon 2 is inflated.

A diaphragm type liquid agitator 8 is disposed together with the liquid supply device 6, to reciprocate the liquid in the balloon 2 inflated by the supplied liquid between the inside of the balloon 2 and the liquid supply passage 6A, for thereby agitating the liquid in the balloon 2. If this agitator 8 is actuated, the liquid in the balloon 2 can be agitated. The liquid portions different in temperature in the balloon 2 are mixed to uniform the liquid temperature in the balloon 2. As a result, the heating irregularity of the liquid in the balloon 2 by high frequency dielectric heating and Joule heating can be lessened.

In the balloon catheter 1, a temperature sensor 9 is disposed in the balloon 2, and temperature information deriving leads 11 (Fig. 4) for deriving the temperature information detected by the temperature sensor 9 are provided. The temperature information deriving leads 11 are connected with the high-frequency power supply apparatus 10 containing a temperature information processor. In this constitution, the high-frequency power supplied from the high-frequency power supply apparatus 10 to the first electrode 5A and the second electrode 5B is quantitatively adjusted.in response to the measurement result of the temperature sensor 9.

It is preferred that the frequency of the high-frequency power is 100 KHz to 2.45 GHz. While the heating by high frequency dielectric heating and Joule heating is carried out, the heating temperature is detected by the temperature sensor 9 disposed in the balloon 2 and fed back to the high-frequency power supply apparatus 10, and the high-frequency power supply apparatus 10 supplies the high-frequency power quantitatively adjusted in response to the measurement result of the temperature sensor 9, to control the temperature of the heating by high frequency dielectric heating and Joule heating.

The high-frequency electrodes 5A and 5B are supported by the support 3B fixed to the outer cylindrical shaft 3 to which the rear end 2R of the balloon 2 is attached. The temperature sensor 9 is fixed to the high-frequency electrode 5A or 5B. In this constitution, the installation positions of the high-frequency electrodes 5A and 5B and the temperature sensor 9 in the balloon 2 are stabilized.

The temperature sensor 9 can be, for example, a thermocouple, but it is not limited to a thermocouple. For example, a semiconductor type temperature measuring element can also be used.

As shown in Fig. 4, the temperature information deriving leads 11 for deriving temperature signals from the temperature sensor 9 and the high-frequency power supply leads 12A and 12B for supplying high-frequency power to the high-frequency electrodes 5A and 5B are respectively covered with an electrically insulating protective covering 13 or 14. The leads are passed through the clearance formed between the outer cylindrical shaft 3 and the inner cylindrical shaft 4.

Since the leads are respectively covered with an electrically insulating protective covering, it does not happen that the leads are short-circuited with each other. In addition, the leak and invasion of high-frequency power are inhibited. This constitution inhibits the heat generation of the outer cylindrical shaft 3 and the inner cylindrical shaft 4 otherwise caused by the leak and invasion of high-frequency power. As a result, the balloon catheter 1 is not required to have a forced cooling mechanism. However, as required, a forced cooling mechanism can also be disposed in the balloon catheter 1.

The material of the temperature information deriving leads 11 and the high-frequency power supply leads 12A and 12B can be wires of copper, silver, platinum, tungsten, alloy, etc.

Particular examples of the material of the electrically insulating protective coverings 13 and 14 include fluorine-based polymers such as polytetrafluoroethylene (PTFE) and tetrafluoroethylene-hexafluoropropylene copolymer (FEP), polyethylene, polypropylene, polyimide resins, polyamide resins, etc.

In the balloon catheter 1, the conductors used to form the high-frequency power supply leads 12A and 12B and the conductors used to form the coils of the high-frequency electrodes 5A and 5B are identical with each other. However, differently manufactured high-frequency power supply leads 12A and 12B can also be connected with the high-frequency electrodes 5A and 5B.

In the balloon catheter 1, to the front end 3F of the outer cylindrical shaft 3, a radiation shielding metallic pipe 3A is attached, and to the front end 4F of the inner cylindrical shaft 4, a radiation shielding metallic pipe 4A is attached. The front end 2F of the balloon 2 is attached to the metallic pipe 4A and fixed to the front end 4F of the inner cylindrical shaft 4. The rear end 2R of the balloon 2 is attached to the metallic pipe 3A and fixed to the front end 3F of the outer cylindrical shaft 3. Since the radiation shielding metallic pipes 3A and 4A are disposed, in the case of fluoroscopy, the radiation shielding metallic pipes 3A and 4A appear on a fluoroscopic image so that the position of the balloon 2 in a patient' s body can be accurately identified. The material of the radiation shielding metallic pipes 3A and 4A can be gold, platinum, stainless steel, etc.

The balloon catheter 1 has the potential detecting electrodes 19A for detecting the potentials around the therapeutic ablation site, attached to the surface of the inner cylindrical shaft 4 at the front end of the inner cylindrical shaft 4, and the potential information deriving leads 20A connected with the potential detecting electrodes 19A and connected with the electrocardiograph 21, passing through the clearance between the outer cylindrical shaft 3 and the inner cylindrical shaft 4.

Furthermore, the balloon catheter 1 has the potential detecting electrodes 19B for detecting the potentials around the therapeutic ablation site, attached to the surface of the outer cylindrical shaft 3 at the front end of the outer cylindrical shaft 3, and the potential information deriving leads 20B connected with the potential detecting electrodes 19B and connected with the electrocardiograph 21, passing through the clearance between the outer cylindrical shaft 3 and the inner cylindrical shaft 4.

The balloon catheter 1 has two potential detecting electrodes 19A disposed with a clearance kept between them and two potential detecting electrodes 19B disposed with a clearance kept between them. However, one or three or more potential detecting electrodes 19A and one or three or more potential detecting electrodes 19B can also be used.

Each of the potential detecting electrodes 19A is formed as a short cylinder with a height (length) of about 1 mm. At the front end 4F of the inner cylindrical shaft 4, a synthetic resin pipe 15 is connected with the tip of the radiation shielding metallic pipe 4A. The potential detecting electrodes 19A are directly tightly fitted around the synthetic resin pipe 15. Each of the potential detecting electrodes 19B is also formed as a short cylinder with a height (length) of about 1 mm. The potential detecting electrodes 19B are directly fitted around the outer cylindrical shaft 3. The material of the potential detecting electrodes 19A and 19B can be platinum, silver, silver plated copper, etc.

The potential information deriving leads 20A and 20B are respectively covered with an electrically insulating protective covering 18 as shown in Fig. 4. These leads pass through the clearance between the outer cylindrical shaft 3 and the inner cylindrical shaft 4 and are connected with the electrocardiograph 21. The leads 20A and 20B can also pass through the slender holes formed in the wall of at least either the outer cylindrical shaft 3 or the inner cylindrical shaft 4. In this case, if the wall of the shaft 3 or 4 electrically insulates the leads 20A and 20B, it is not necessary to cover the leads 20A and 20B with an electrically insulating protective covering 18.

For checking the potentials detected by the potential detecting electrodes 19A and 19B, as shown in Fig. 1, the potential information deriving leads 20A and 20B are connected with an ordinary electrocardiograph 21, and the chart of the detected potentials by the electrocardiograph 21 is displayed on the monitor screen or printed out.

How to use the balloon catheter 1 is explained below in reference to a case where circumferential ablation is performed at the pulmonary vein ostia of the heart.

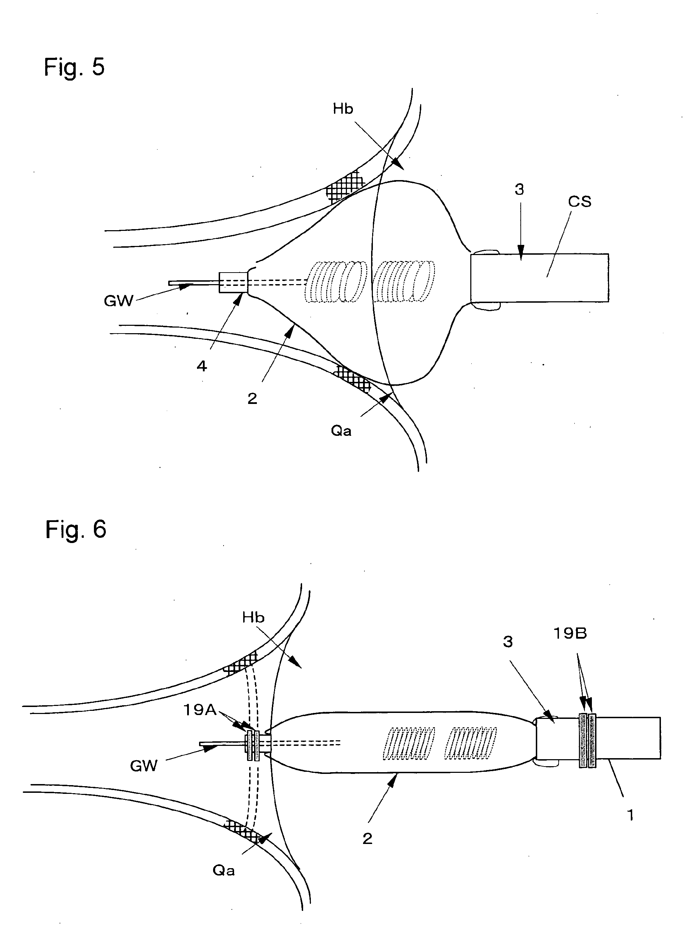

As shown in Fig. 5, the balloon 2 as deflated is pressed by the catheter shaft CS along the guide wire GW inserted percutaneously into a patient's body beforehand, while it goes from the inferior vena cava QA into the left atrium Ha and further goes through the interatrial septum Hw into the right atrium Hb. Subsequently, a liquid is supplied into the balloon 2, to inflate the balloon 2, applying and keeping it to and in contact with the circumference of a pulmonary vein ostium Qa. Then, high-frequency power is supplied across the high-frequency electrodes 5A and 5B in the balloon 2. As a result, the circumference of the pulmonary vein ostium Qa is heated to perform ablation. The circumferential ablation of the remaining three pulmonary vein ostia is also similarly performed.

After the circumferential ablation of a pulmonary vein opening is completed, the potential information from the potential detecting electrodes 19A and 19B is read on the electrocardiograph 21. Based on the read result, whether the ablation is acceptable is judged.

In the case where the potential detecting electrodes 19A are used, as shown in Fig. 6, the balloon catheter (balloon ablation catheter) 1 is kept inserted, and the potential detecting electrodes 19A are positioned near the therapeutic ablation site (for example, the inner surface of the atrium). The potential information from this position is sent through the potential information deriving leads 20A to the electrocardiograph 21. The result is shown on the chart of the electrocardiograph 21. In reference to the detection result displayed on the chart, whether or not the ablation is acceptable is judged. If the result of judgment is unacceptable, the balloon 2 is inflated again to repeat the ablation process. Meanwhile, Fig. 6 shows a case where the balloon 2 is deflated after completion of the first ablation process.

Also in the case where the potential detecting electrodes 19B are used, as shown in Fig. 7, the balloon catheter (balloon ablation catheter) 1 is kept inserted, and the potential detecting electrodes 19B are positioned near the therapeutic ablation site (for example, the inner surface of the atrium). The potential information from this position is sent through the potential information deriving leads 20B to the electrocardiograph 21. The result is displayed on the chart of the electrocardiograph 21. From the detection result displayed on the chart, whether or not the ablation is acceptable is judged. If the result of judgment is unacceptable, the balloon 2 is inflated again to repeat the ablation process. Meanwhile, Fig. 7 shows a case where the balloon 2 is deflated after completion of the first ablation process.

Depending on the sites at which the potentials are detected, the potential detecting electrodes 19A and 19B can be simultaneously actuated to detect the potentials of two sites simultaneously, for checking the respective detection results.

If the results of all the ablation processes performed are judged to be acceptable, the balloon catheter (balloon ablation catheter) 1 is removed from the body, to complete the medical procedure.

Embodiments of the balloon catheter (balloon ablation catheter) of the invention are explained below as examples and comparative examples.

### Example 1

A balloon 2 formed like a tapered cone with a length of 30 mm from the front end to the rear end of the balloon 2, with the largest outer diameter of 30 mm on the rear end side and a film thickness of 160 µm was produced as described below.

A balloon glass mold having a surface corresponding to a desired balloon form was dipped in 13% polyurethane solution, and the coated mold was heated to evaporate the solvent, to form a urethane polymer film on the surface of the mold as the balloon 2 by a dipping method.

As the outer cylindrical shaft 3 of the catheter 1, a 30% barium sulfate-containing PVC tube with an outer diameter of 12Fr, an inner diameter of 2.7 mm and an overall length of 800 mm was prepared. As the metallic pipe 3A, a stainless steel pipe with a diameter of 2.8 mm and a length of 7 mm, with its outer surface finished by sandblasting, was prepared. The metallic pipe 3A was partially inserted and fitted into the front end of the outer cylindrical shaft 3, and a nylon yarn with a diameter of 0.1 mm was used to bind and fix them. Two electrodes respectively with an outer diameter of 4.0 mm, an inner diameter of 3.8 mm and a width of 1 mm were fitted around the outer cylindrical shaft 3 at the front end of the outer cylindrical shaft 3 with a clearance of 1 mm kept between them, and fixed using an adhesive, to form the potential detecting electrodes 19B. The potential information deriving leads 20B respectively covered with an electrically insulating protective covering were passed through the outer cylindrical shaft 3 at the portion covered with the potential detecting electrodes 19B, and connected with the potential detecting electrodes 19B. The four-way connector 7 was fitted around the outer cylindrical shaft 3 at the proximal end of the outer cylindrical shaft 3, and a nylon yarn with a diameter of 0.1 mm was used to bind and fix them.

On the other hand, as the inner cylindrical shaft 4 of the catheter 1, a nylon 11 tube having an outer diameter of 4Fr, an inner diameter of 1.1 mm and an overall length of 900 mm was prepared. As the metallic pipe 4A, a stainless steel pipe with a diameter of 1.2 mm and a length of 6 mm, with its outer surface finished by sandblasting, was prepared. The metallic pipe 4A was partially inserted and fitted into the distal end of the inner cylindrical shaft 4, and a nylon yarn with a diameter of 0.1 mm was used to bind and fix them. A synthetic resin pipe 15 with an outer diameter of 2.0 mm, an inner diameter of 1.1 mm and a length of about 10 mm was partially fitted around the metallic pipe 4A and bonded as an additional part. Two electrodes respectively with an outer diameter of 2.5 mm, an inner diameter of 2.0 mm and a width of 1 mm were fitted around the synthetic resin pipe 15 at the front end of the synthetic resin pipe 15 with a clearance of 1 mm kept between them, and fixed using an adhesive, to form the potential detecting electrodes 19A. The potential information deriving leads 20A respectively covered with an electrically insulating protective covering were connected with the potential detecting electrodes 19A. While the potential information deriving leads 20A and 20B were drawn out on the rear end side of the catheter 1, the inner cylindrical shaft 4 was inserted through the inner cylindrical shaft through-hole of the four-way connector 7. The cap of the four-way connector 7 was tightened to complete a double cylindrical catheter 1.

An insulated annealed copper wire plated with 0.1 µm of silver with a diameter of 0.5 mm was formed at its tip portion as a coil with an inner diameter of 1.6 mm and with a length of 10 mm in the axial direction of the catheter 1 (i.e., a width of 10 mm), as each of the high-frequency electrodes 5A and 5B. Tetrafluoroethylene-hexafluoropropylene copolymer (FEP) was used to cover the portion other than the coil, to form an electrically insulating protective covering 14. In this way, the high-frequency power supply leads 12A and 12B provided with the high-frequency electrodes 5A and 5B were prepared.

As the temperature sensor 9, an extra fine copper-constantan thermocouple double wire was prepared. The wire was covered with an electrically insulating protective covering 13 of polytetrafluoroethylene. Thus, a temperature sensor 9 with temperature information deriving leads 11 was manufactured.

The temperature sensor 9 was fixed to the high-frequency electrode 5A, and subsequently the high-frequency electrodes 5A and 5B were fitted around the inner cylindrical shaft 4 at the front end of the inner cylindrical shaft 4. Then, the temperature information deriving leads 11 and the high-frequency power supply leads 12A and 12B were passed through the clearance between the outer cylindrical shaft 3 and the inner cylindrical shaft 4, and the rear ends of the temperature information deriving leads 11 and the high-frequency power supply leads 12A and 12B were pulled out of the four-way connector 7. Furthermore, the front ends of the temperature information deriving leads 11 and the high-frequency power supply leads 12A and 12B were fixed to the metallic pipe 3A using an aramid fiber fastener with the distance between the high-frequency electrodes 5A and 5B kept at 2 mm.

When the high-frequency electrodes 5A and 5B were fixed, a polypropylene pipe (with a length of 2 mm in the axial direction) was fitted around the inner cylindrical shaft 4 as the spacer 17 lest the shortest distance Esd between the high-frequency electrodes 5A and 5B should be less than 1 mm.

Finally the front end 2F of the balloon 2 was bound and fixed to the metallic pipe 4A using a nylon yarn with a diameter of 0. 1 mm, and the rear end 2R of the balloon 2 was bound and fixed to the metallic pipe 3A using a nylon yarn with a diameter of 0.1 mm.

Thus, a balloon catheter (balloon ablation catheter) 1 was completed. This catheter is hereinafter called the ablation catheter of Example 1.

Heat generation test of metallic guide wires:
The metallic guide wires of the ablation catheter of Example 1 and a conventional ablation catheter were compared in heat generation.

### Comparative Example 1

At first, a metallic guide wire was used in a conventional ablation catheter to examine the heat generation of the metallic guide wire.

As the conventional ablation catheter, a catheter identical with the catheter 1 of Fig. 1 except that one high-frequency electrode 5B was removed, was prepared. This catheter is hereinafter called the ablation catheter of Comparative Example 1. As the counter electrode plate 54 (Fig. 8), an aluminum sheet with a vertical length of 7. 5 cm, a horizontal length of 15 cm and a thickness of 100 µm was prepared.

The ablation catheter of Comparative Example 1 was immersed in a water tank filled with 37°C physiological saline. The high-frequency power supply lead 12A was connected with the high-frequency power supply apparatus 10. The counter electrode plate 54 was disposed on the outer wall surface of the water tank and connected with the high-frequency power supply apparatus 10. Into the balloon 2, a liquid obtained by diluting a contrast medium (ioxaglic acid injection: trade name Hexabrix 320) to 50% using physiological saline was injected to inflate the balloon 2 to such a state that the largest outer diameter on,the rear end side of the balloon 2 became 30 mm.

As the guide wire, a SUS304 wire with a diameter of 0.025 inch (about 0.6 mm) and a length of 1500 mm was used. The guide wire was inserted into the inner cylindrical shaft 4 of the ablation catheter of Comparative Example 1, and with the front end of the guide wire projected by about 1 cm from the front end of the catheter, a thermocouple was stuck to the front end of the guide wire.

With the frequency of the high-frequency power supply apparatus 10 set at 13.56 MHz and with the temperature in the balloon 2 set at 70°C, high-frequency power was supplied for 5 minutes. As a result, about 60 seconds after start of power supply, the temperature at the front end of the guide wire rose up to 50°C, and thereafter the temperature lingered at about 50°C (50°C±3°C) .

From this experiment of performing ablation using the ablation catheter of Comparative Example 1, it can be estimated that the high-frequency power supply caused the high-frequency electric current to flow to the metallic guide wire, to also heat the metallic guide wire.

### Example 2

A catheter identical with the ablation catheter of Example 1 except that a metallic guide wire was inserted through the hollow portion of the inner cylindrical shaft 4, was prepared. This catheter is hereinafter called the ablation catheter of Example 2. The heat generation of the metallic guide wire used in the ablation catheter of Example 2 was examined.

The ablation catheter of Example 2 was immersed in a water tank filled with 37°C physiological saline. The high-frequency power supply leads 12A and 12B were connected with the high-frequency power supply apparatus 10. Into the balloon 2, a liquid obtained by diluting a contrast medium (ioxaglic acid injection: trade name Hexabrix 320) to 50% using physiological saline was injected to inflate the balloon 2 to such a state that the largest outer diameter on the rear end side of the balloon 2 became 30 mm.

As the guide wire, a SUS304 wire having a diameter of 0.025 inch (about 0.6 mm) and a length of 1500 mm was used. The guide wire was inserted into the inner cylindrical shaft 4 of the ablation catheter of Example 2, and with the front end of the guide wire projected by about 1 cm from the front end of the catheter, a thermocouple was stuck to the front end of the guide wire.

With the frequency of the high-frequency power supply apparatus 10 set at 13.56 MHz and with the temperature in the balloon 2 set at 75°C, high-frequency power was supplied for 5 minutes. As a result, even when 5 minutes passed after start of power supply, the temperature at the front end of the guide wire was kept at about 40°C (40°C±3°C).

From this experiment of performing ablation using the ablation catheter of Example 2, it can be estimated that since both the high-frequency electrodes were disposed in the balloon 2 made of an electrically highly resistant material, high-frequency current did not flow to the metallic guide wire during ablation, and therefore that the ablation of a blood vessel or tissue other than the target lesion site otherwise caused by the heating of the metallic guide wire did not occur.

Examination on the surface area SA of the first high-frequency electrode 5A and the surface area SB of the second high-frequency electrode 5B:

### Comparative Example 2

A catheter identical with the ablation catheter of Example 1 except that the lengths of the high-frequency electrodes 5A and 5B in the axial direction of the catheter were 0.5 mm respectively, was prepared. The surface areas SA and SB of the high-frequency electrodes 5A and 5B of this catheter were about 10 mm² respectively. This catheter is hereinafter called the ablation catheter of Comparative Example 2.

### Example 3

A catheter identical with the ablation catheter of Example 1 except that the lengths of the high-frequency electrodes 5A and 5B in the axial direction of the catheter 1 were 1 mm respectively, was prepared. The surface areas of SA and SB of the high-frequency electrodes 5A and 5B in this catheter were about 20 mm² respectively. This catheter is hereinafter called the ablation catheter of Example 3.

The ablation catheters of Comparative Example 2 and Examples 1 and 3 were respectively immersed in a water tank filled with 37°C physiological saline, and in each of the catheters, the high-frequency power supply leads 12A and 12B were connected with the high-frequency power supply apparatus 10. In each of the catheters, a liquid obtained by diluting a contrast medium (ioxaglic acid injection: trade name Hexabrix 320) to 50% using physiological saline was injected into the balloon 2, to inflate the balloon 2 to such a state that the largest outer diameter on the rear end side of the balloon became 30 mm.

With the frequency of the high-frequency power supply apparatus 10 set at 13.56 MHz and with the temperature in the balloon 2 set at 75°C, high-frequency power was supplied for 5 minutes.

As a result, in the ablation catheter of Comparative Example 2, since the surface areas of the high-frequency electrodes were small, the high-frequency electrical current concentrated, and only the areas around the high-frequency electrodes 5A and 5B reached a temperature of 100°C. So, it was observed that the liquid near the electrodes in the balloon 2 boiled and bubbled. Such a high temperature as to cause boiling in a patient' s body is not preferred for the patient. Furthermore, since boiling occurred, the impedance between the electrodes violently changed, and it was difficult to achieve the impedance matching with the high-frequency power supply apparatus.

On the contrary, in the ablation catheter of Example 3, liquid boiling was not observed. Furthermore, in the ablation catheter of Example 1 either, liquid boiling was not observed. It is necessary that the surface areas of the high-frequency electrodes 5A and 5B are 20 mm² or more respectively, in which case no boiling can be observed.

In the ablation catheter of Example 3, the surface temperature of the balloon 2 rose only to about 50°C, but in the ablation catheter of Example 1, the surface temperature of the balloon 2 rose to about 60°C. The reason is that in the ablation catheter of Example 3 compared with the ablation catheter of Example 1, since the surface areas of the high-frequency electrodes were smaller, the high-frequency electrical current more concentrated, causing the areas near the high-frequency electrodes 5A and 5B only to reach 75°C.

In the ablation catheter of Example 3, the necessity of setting the temperature in the balloon 2 at 90°C for keeping the surface temperature of the balloon 2 at 60°C was confirmed. In view of safety, it is desirable that the highest temperature reached in a patient's body is lower. The ablation catheter of Example 1 is considered to be more excellent than the ablation catheter of Example 3 in view of safety.

Examination on the shortest distance Esd between high-frequency electrodes:

### Comparative Example 3

A catheter identical with the ablation catheter of Example 1 except that the distance between the high-frequency electrodes 5A and 5B was 0.5 mm, was prepared. This catheter is hereinafter called the ablation catheter of Comparative Example 3.

### Example 4

A catheter identical with the ablation catheter of Example 1 except that the distance between the high-frequency electrodes 5A and 5B was 1 mm, was prepared. This catheter is hereinafter called the ablation catheter of Example 4.

The ablation catheters of Comparative Example 3 and Examples 1 and 4 were immersed in a water tank filled with 37°C physiological saline, and in each of the catheters, the high-frequency power supply leads 12A and 12B were connected with the high-frequency power supply apparatus 10. In each of the catheters, a liquid obtained by diluting a contrast medium (ioxaglic acid injection: trade name Hexabrix 320) to 50% using physiological saline was injected into the balloon 2, to inflate the balloon 2 to such a state that the largest outer diameter on the rear end side of the balloon 2 became 30 mm.

With the frequency of the high-frequency power supply apparatus 10 set at 13.56 MHz and with the temperature in the balloon 2 set at 75°C, high-frequency power was supplied for 5 minutes.

As a result, in the ablation catheter of Comparative Example 3, the high-frequency electrical current concentrated and the temperature in the areas near the high-frequency electrodes 5A and 5B (especially on the side where the high-frequency electrodes were close to each other) reached 100°C, since the shortest distance Esd between the high-frequency electrodes was short though the surface areas of the high-frequency electrodes were as large as 200 mm² respectively. So, it was observed that the liquid near the electrodes in the balloon 2 boiled and bubbled. Such a high temperature as to cause boiling in a patient's body is not preferred for the patient. Furthermore, since boiling occurred, the impedance between the electrodes violently changed, and it was difficult to achieve impedance matching with the high-frequency power supply apparatus.

On the contrary, in the ablation catheter of Example 4, liquid boiling was not observed. Furthermore, either in the ablation catheter of Example 1, liquid boiling was not observed. It is preferred that the shortest distance between the high-frequency electrodes 5A and 5B is 1 mm or more, in which case boiling is not observed.

Examination on the effectiveness of spacer:

### Comparative Example 4

An ablation catheter identical with the ablation catheter of Example 1 in which the space 17 was removed, was prepared. In this catheter, the distance between the high-frequency electrodes 5A and 5B could freely change. This catheter is hereinafter called the ablation catheter of Comparative Example 4.

The ablation catheters of Comparative Example 4 and Example 1 were immersed in a water tank filled with 37°C physiological saline, and in each of the catheters, the high-frequency power supply leads 12A and 12B were connected with the high-frequency power supply apparatus 10. In each of the catheters, a liquid obtained by diluting a contrast medium (ioxaglic acid injection: trade name Hexabrix 320) to 50% using physiological saline was injected into the balloon 2, to inflate the balloon 2 to such a state that the largest outer diameter on the rear end side of the balloon 2 became 30 mm.

With the frequency of the high-frequency power supply apparatus 10 set at 13.56 MHz and with the temperature in the balloon 2 set at 75°C, high-frequency power was supplied for 5 minutes.

The shortest distance Esd between the high-frequency electrodes 5A and 5B in the ablation catheter of Comparative Example 4 was changed to 2 mm, 0.5 mm and 0 mm (short-circuit).

As a result, when the distance between the high-frequency electrodes 5A and 5B was 2 mm, liquid boiling was not observed. When the distance between the high-frequency electrodes 5A and 5B was 0.5 mm, it was observed the liquid near the electrodes boiled and bubbled. When the distance between the high-frequency electrodes 5A and 5B was 0 mm (short-circuit), the balloon 2 was not heated. Furthermore, the high-frequency power supply leads 12A and 12B generated heat.

From the above results, it was found that if the shortest distance Esd between the high-frequency electrodes 5A and 5B is too short without the spacer 17 disposed, the liquid near the high-frequency electrodes boiled or that the high-frequency electrodes were short-circuited with each other, not allowing heating. It is preferred to dispose the spacer 17 for reliably maintaining the shortest distance Esd between the high-frequency electrodes 5A and 5B.

Test for detecting the potentials at ablation site:

### Example 5

A potential detecting test was performed to check the potential detecting functions of the potential detecting electrodes 19A and 19B in the ablation catheter of Example 1.

A subject (pig) to be used for the potential detecting test was prearranged beforehand, and the potential information deriving leads 20A and 20B were connected with the electrocardiograph 21.

At first, the potential detecting electrodes 19A were applied to the body surface of the subject near the heart, to record the detected potentials on the chart of the electrocardiograph 21. Then, the potential detecting electrodes 19B were applied to the body surface of the subject near the heart, to record the detected potentials on the chart of the electrocardiograph 21.
All the recorded results on the charts were normal.

In this potential detecting test, the potentials of the body surface of the subject were detected. If the potentials of the body surface of the subject can be normally detected, the potentials at the ablation site in the body of the subject can also be normally detected. Thus, it was confirmed that both the potential detecting electrodes 19A and 19B can adequately detect the potentials in a patient's body.

Heat generation test of potential detecting electrodes:

### Example 5

A catheter identical with the ablation catheter of Comparative Example 1 except that potential detecting electrodes were disposed, was prepared. This catheter is hereinafter called the ablation catheter of Comparative Example 5. As the counter electrode plate 54 (Fig. 8), the same counter electrode as described for Comparative Example 1 was used.

The ablation catheter of Comparative Example 5 was immersed in a water tank filled with 37°C physiological saline, and the high-frequency power supply lead wire 12A was connected with the high-frequency power supply apparatus 10. The counter electrode 54 was disposed on the outer wall surface of the water tank and connected with the high-frequency power supply apparatus 10. Into the balloon 2, a liquid obtained by diluting a contrast medium (ioxaglic acid injection: trade name Hexabrix 320) to 50% by physiological saline was injected to inflate the balloon 2 to such a state that the largest outer diameter on the rear end side of the balloon 2 became 30 mm.

With the frequency of the high-frequency power supply apparatus 10 set at 13.56 MHz and with the temperature in the balloon 2 set at 70°C, high-frequency power was supplied for 5 minutes. A thermocouple was stuck to right above the potential detecting electrodes 19B, to measure the temperature. As a result, in about 30 seconds after start of power supply, the temperature of the potential detecting electrodes 19B rose to 60°C, and also thereafter, the temperature was kept at about 60°C (60°C±3°C).

From the above, in the ablation using the ablation catheter of Comparative Example 5, it can be estimated that when high-frequency power was supplied, the high-frequency electrical current flowed to the potential detecting electrodes, to also heat the potential detecting electrodes.

### Example 6

The heat generation by the potential detecting electrodes in the ablation catheter of Example 1 was examined.

The ablation catheter of Example 1 was immersed in a water tank filled with 37°C physiological saline. The high-frequency power supply leads 12A and 12B were connected with the high-frequency power supply apparatus 10. Into the balloon 2, a liquid obtained by diluting a contrast medium (ioxaglic acid injection: trade name Hexabrix 320) to 50% using physiological saline was injected to inflate the balloon 2 to such a state that the largest outer diameter on the rear end side of the balloon 2 became 30 mm.

With the frequency of the high-frequency power supply apparatus 10 set at 13.56 MHz and with the temperature in the balloon 2 set at 75°C, high-frequency power was supplied for 5 minutes. A thermocouple was stuck to right above the potential detecting electrodes 19B, to measure the temperature. As a result, even when 5 minutes passed after start of power supply, the temperature of the potential detecting electrodes was about 40°C (40°C±3°C).

From the above, in the ablation using the ablation catheter of Example 1, since both the high-frequency electrodes were disposed in the balloon 2 made of an electrically highly resistant material, it can be estimated that since the high-frequency electrical current did not flow to the potential detecting electrodes during ablation, the ablation of a blood vessel or tissue other than the target lesion site by the heating of the potential detecting electrodes did not happen.

This invention is not limited to or by the above examples, and can also be carried out in the following mode.

For example, the ablation catheter of Example 1 comprises the liquid supply device 6, the high-frequency power supply apparatus 10 and the electrocardiograph 21. However, since the liquid supply device 6, the high-frequency power supply apparatus 10 and the electrocardiograph 21 are available separately and can be connected with the catheter 1 for actual treatment, the balloon catheter (balloon ablation catheter) of the invention is not required to comprise the liquid supply device 6, the high-frequency current source 10 or the electrocardiograph 21. Industrial applicability

In the balloon catheter of the invention, high-frequency electric current flows between the electrodes positioned to face each other with a clearance kept between them in a balloon, to heat the liquid in the balloon, and the heat is used to perform the ablation of the organism tissue kept in contact with the balloon. The surface areas of the electrodes are 20 mm² or more respectively, and the potential detecting electrodes for detecting the potentials of the ablation site are disposed outside the balloon at least on the front or rear side of the balloon. In the balloon catheter of the invention, since the counter electrode plate required in the conventional catheter is not necessary, there is no problem of heat generated by it, and the heat generation of the guide wire and the heat generation of the potential detecting electrodes are inhibited. So, the invention provides a balloon ablation catheter safer for a patient and capable of reducing the burden of catheter invasion on the patient.

## Claims

1. A balloon catheter comprising a catheter shaft, a balloon attached to the catheter shaft, a first electrode and a second electrode positioned in the balloon with a clearance kept between them along the catheter shaft, high-frequency power supply leads for supplying high-frequency power to the first and second electrodes, and a liquid supply passage for supplying a liquid into the balloon, wherein the surface area SA of the first electrode and the surface area SB of the second electrode are 20 mm² or more respectively.

2. A balloon catheter, according to claim 1, wherein the shortest distance Esd between the first electrode and the second electrode is 1 mm or more.

3. A balloon catheter, according to claim 1, wherein a spacer for keeping the clearance between the first electrode and the second electrode is disposed between these electrodes.

4. A balloon catheter, according to claim 1, which further comprises a temperature sensor disposed inside or on the outer surface of the balloon, and temperature information deriving leads for deriving the temperature information detected by the temperature sensor.

5. A balloon catheter comprising a catheter shaft, a balloon attached to the catheter shaft, a first electrode and a second electrode positioned in the balloon with a clearance kept between them along the catheter shaft, high-frequency power supply leads for supplying high-frequency power to the first and second electrodes, and a liquid supply passage for supplying a liquid into the balloon, wherein potential detecting electrodes for detecting the potentials of the therapeutic site are disposed on the catheter shaft outside the balloon on the front end side or rear end side of the catheter shaft, and potential information deriving leads for deriving the potential information detected by the potential detecting electrodes are provided.

6. A balloon catheter, according to claim 5, wherein the surface area SA of the first electrode and the surface area SB of the second electrode are 20 mm² or more respectively.

7. A balloon catheter, according to claim 5, wherein the shortest distance Esd between the first electrode and the second electrode is 1 mm or more.

8. A balloon catheter, according to claim 5, wherein a spacer for keeping the clearance between the first electrode and the second electrode is disposed between these electrodes.

9. A balloon catheter, according to claim 5, which further comprises a temperature sensor disposed inside or on the outer surface of the balloon, and temperature information deriving leads for deriving the temperature information detected by the temperature sensor.

10. A balloon catheter, according to claim 1, wherein the catheter shaft comprises an outer cylindrical shaft and an inner cylindrical shaft provided in the outer cylindrical shaft movably along the outer cylindrical shaft; the front end of the balloon is fixed to the front end of the inner cylindrical shaft while the rear end of the balloon is fixed to the front end of the outer cylindrical shaft, so that when the inner cylindrical shaft is moved relatively to the outer cylindrical shaft, the balloon can be deformed; and the first and second electrodes are positioned with a clearance kept between them along the inner cylindrical shaft.

11. A balloon catheter, according to claim 10, wherein the liquid supply passage is formed as the clearance between the outer cylindrical shaft and the inner cylindrical shaft.

12. A balloon catheter, according to claim 5, wherein the catheter shaft comprises an outer cylindrical shaft and an inner cylindrical shaft provided in the outer cylindrical shaft movably along the outer cylindrical shaft; the front end of the balloon is fixed to the front end of the inner cylindrical shaft while the rear end of the balloon is fixed to the front end of the outer cylindrical shaft, so that when the inner cylindrical shaft is moved relatively to the outer cylindrical shaft, the balloon can be deformed; the first and second electrodes are positioned with a clearance kept between them along the inner cylindrical shaft; in the case where the potential detecting electrodes are positioned outside the balloon on the front end side of the catheter shaft, the potential detecting electrodes are disposed on the inner cylindrical shaft; and in the case where the potential detecting electrodes are positioned outside the balloon on the rear end side of the catheter shaft, the potential detecting electrodes are disposed on the outer cylindrical shaft.

13. A balloon catheter, according to claim 12, wherein the liquid supply passage is formed as the clearance between the outer cylindrical shaft and the inner cylindrical shaft.

14. A balloon catheter, according to claim 4, wherein a temperature information processor connected with the temperature information deriving leads and a high-frequency power adjusting device connected with the high-frequency power supply leads are provided to ensure that the high-frequency power supplied to the first and second electrodes can be adjusted by the high-frequency power adjusting device in response to the temperature judged by the temperature information processor.

15. A balloon catheter, according to claim 9, wherein a temperature information processor connected with the temperature information deriving leads and a high-frequency power adjusting device connected with the high-frequency power supply leads are provided to ensure that the high-frequency power supplied to the first and second electrodes can be adjusted by the high-frequency power adjusting device in response to the temperature judged by the temperature information processor.

16. A balloon catheter, according to claim 1, wherein the frequency of the high-frequency power supplied to the first and second electrodes is 100 KHz to 2.45 GHz; and the high-frequency power heats the liquid supplied from the liquid supply passage into the balloon for filling the balloon, to a temperature of 50°C to 80°C.

17. A balloon catheter, according to claim 5, wherein the frequency of the high-frequency power supplied to the first and second electrodes is 100 KHz to 2.45 GHz; and the high-frequency power heats the liquid supplied from the liquid supply passage into the balloon for filling the balloon, to a temperature of 50°C to 80°C.

18. A balloon catheter, according to claim 1, wherein a liquid agitator connected with the liquid supply passage is provided to ensure that the liquid supplied from the liquid supply passage into the balloon for filling the balloon can be reciprocated between the liquid supply passage and the inside of the balloon, so that the liquid can be agitated in the balloon.

19. A balloon catheter, according to claim 5, wherein a liquid agitator connected with the liquid supply passage is provided to ensure that the liquid supplied from the liquid supply passage into the balloon for filling the balloon can be reciprocated between the liquid supply passage and the inside of the balloon, so that the liquid can be agitated in the balloon.
